# DEMANDE DE BREVET EUROPEEN

(11) **EP 1 378 213 A1**
(43) Date de publication de la demande: **07.01.2004**
(21) Numéro de dépôt: 03291565.4
(22) Date de dépôt: 25.06.2003
(51) Int. Cl.: A61F 2/08

(54) **Vis d'interference bioresorbable**

(30) Priorité: 03.07.2002 FR 0208321
(71) Demandeur: PHUSIS, 38330 Saint Ismier (FR)
(72) Inventeur: Huet-Olivier, Jacqueline, 38330 Saint-Ismier (FR); Boileau, Pascal, 06200 Nice (FR)
(74) Mandataire: Geismar, Thierry

(57) **Abrégé**

L'invention concerne une vis d'interférence (1), notamment destinée à la fixation de tendons à l'intérieur d'un tunnel trans-osseux, ladite vis (1) étant réalisée en matériau biorésorbable et étant constituée d'un corps (2) monobloc pourvu d'une surface externe (4) sur laquelle est prévu un filet (5) de vis, ladite vis (1) comprenant une première zone (6) dite proximale de diamètre D1, une deuxième zone (7) dite distale de diamètre D2 et une zone intermédiaire (8) prévue entre lesdites zones proximale (6) et distale (7), ladite vis (1) étant caractérisée en ce que le diamètre D1 est supérieur au diamètre D2 de sorte à permettre, outre la fixation des tendons, la neutralisation de leur mouvement dans les tunnels trans-osseux.

## Description

L'invention concerne une vis d'interférence réalisée en matériau biorésorbable.

Elle s'applique typiquement à la fixation d'un tissu mou à l'intérieur d'un tunnel trans-osseux.

En particulier, ce type de vis est utilisé dans les deux indications chirurgicales suivantes :
- la refixation de tendons pathologiques, par exemple dans le cadre d'une ténodèse du long biceps dans la chirurgie de l'épaule,
- la fixation de greffons purement tendineux, par exemple dans le cadre de la reconstruction du ligament croisé antérieur par plastie aux tendons ischio-jambiers.

On connaît déjà, notamment du document FR-2 701 386, des vis d'interférence biorésorbables permettant de fixer un transplant dans un tunnel trans-osseux, en particulier lors de la chirurgie du ligament croisé antérieur du genou.

Toutefois, ce type de vis, bien que permettant une fixation très efficace du transplant lorsque celui-ci comporte le tendon rotulien et une petite barrette osseuse à ses deux extrémités, présente un certain nombre d'inconvénients pour la fixation de transplants purement tendineux, c'est-à-dire ne comportant aucun bloc osseux.

En effet, on a observé, par des tests mécaniques de fatigue en traction, des phénomènes de glissement des tendons sous la vis entraînant une détente de la plastie, et ce pour les niveaux de charge qu'un genou en phase de rééducation post-opératoire doit supporter.

En outre, ce type de fixation entraîne des risques de détérioration des fibres tendineuses pouvant entraîner une fragilisation de la plastie.

Ces inconvénients sont dus au fait que les vis sont directement mises en place sur le tendon, et non le long du bloc osseux.

Par ailleurs, ces vis, outre leur fonction de fixation, doivent permettre également de favoriser la consolidation du tendon dans le tunnel trans-osseux, en plaquant le tendon sur la paroi du tunnel.

Toutefois, lorsqu'on utilise une vis de diamètre important, dans le but d'obtenir une fixation plus efficace, la pression élevée exercée par la vis sur la partie du tendon en contact avec le tunnel peut entraîner l'ischémie dudit tendon, retardant ainsi sa vascularisation et sa consolidation.

D'autre part, il est souhaitable de neutraliser les mouvements du tendon à l'intérieur du tunnel trans-osseux. En effet, ces mouvements peuvent entraîner l'élargissement de ce tunnel (ostéolyse).

Pour surmonter ces problèmes, les chirurgiens utilisent souvent deux moyens de fixation : des vis d'interférence pour neutraliser les mouvements du tendon à l'intérieur du tunnel et des moyens de fixation supplémentaires tels que des agrafes ou des ensembles vis/rondelles qui sont positionnés à l'extérieur du tunnel, sur l'os cortical, dans le but d'obtenir une fixation mécaniquement plus performante.

Toutefois cette solution présente des inconvénients non seulement en termes économiques, puisque le nombre d'implants à utiliser est élevé, mais également en termes cliniques, en ce que les moyens de fixation supplémentaires sont généralement mal tolérés et doivent être retirés lors d'une seconde intervention chirurgicale.

Pour pallier ces inconvénients, l'invention propose une vis d'interférence réalisée en matériau biorésorbable, dont la géométrie particulière lui confère à la fois des propriétés de fixation mécanique performante et de neutralisation des mouvements des tissus mous dans les tunnels trans-osseux, ce qui présente des avantages tant économiques que cliniques.

A cet effet, et selon un premier aspect, l'invention propose une vis d'interférence, notamment destinée à la fixation de tendons à l'intérieur d'un tunnel trans-osseux, ladite vis étant réalisée en matériau biorésorbable et étant constituée d'un corps monobloc pourvu d'une surface externe sur laquelle est prévu un filet de vis, ladite vis comprenant une première zone dite proximale de diamètre D1, une deuxième zone dite distale de diamètre D2 et une zone intermédiaire prévue entre lesdites zones proximale et distale, le diamètre D1 étant supérieur au diamètre D2 de sorte à permettre, outre la fixation des greffons, la neutralisation de leur mouvement dans les tunnels trans-osseux.

Selon un mode de réalisation, le diamètre D1 est supérieur au diamètre D2 d'une valeur comprise entre 10% et 20% du diamètre D1.

D'autres objets et avantages de l'invention apparaîtront au cours de la description qui suit, faite en référence aux dessins annexés dans lesquels :

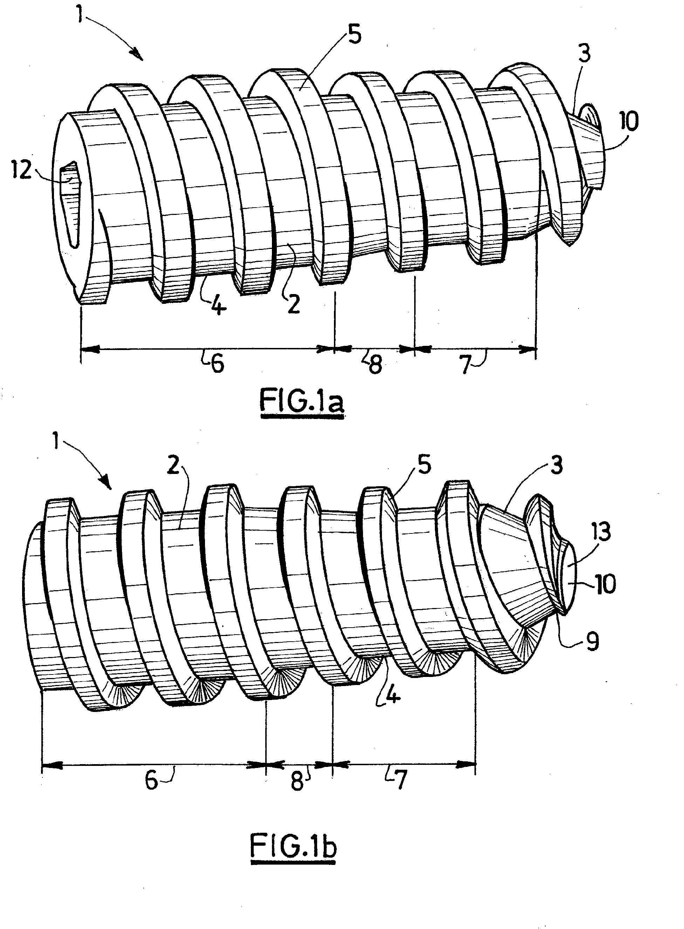

- les figures 1a et 1b sont des vues en perspective de la vis d'interférence, la figure 1a montrant en particulier l'extrémité proximale de la vis, et la figure 1b montrant en particulier son extrémité distale ;
- la figure 2 est une vue longitudinale en coupe d'un premier exemple de vis ;
- les figures 3a et 3b sont des vues agrandies d'une partie du filet prévue respectivement sur la zone proximale et sur la zone distale de la vis de la figure 2 ;
- la figure 4 est une vue suivant la flèche F de la figure 2 ; et
- la figure 5 est une vue longitudinale en coupe d'un deuxième exemple de vis.

En relation avec les figures, on décrit une vis d'interférence 1 réalisée en matériau biorésorbable qui est plus particulièrement destinée à la fixation de tendons à l'intérieur d'un tunnel trans-osseux.

Cette vis 1 est réalisée en une seule pièce notamment par moulage par injection.

Suivant une réalisation, et pour qu'il présente une bonne aptitude au moulage, le matériau biorésorbable comprend un polymère de grande pureté chimique, de masse moléculaire supérieure à environ 250 000 et de faible indice de polydispersité, par exemple inférieur à 2.

Le matériau biorésorbable à partir duquel est réalisée la vis est typiquement un stéréocopolymère d'acide lactique de haute pureté chimique (copolymère des acides L-lactique et D-lactique).

On peut également utiliser l'homopolymère de l'acide L-lactique ou des copolymères biorésorbables de l'acide lactique et d'un comonomère compatible, par exemple dérivé d'α-hydroxyacides, ainsi que les dérivés et/ou mélanges de ces produits, par exemple synthétisés suivant le procédé décrit dans la demande de brevet FR-2 745 005 issue de la demanderesse.

Dans un exemple particulier, le matériau biorésorbable comprend 98% d'acide L-lactique et 2% d'acide D-lactique, sa masse moléculaire moyenne est comprise entre 300 000 et 400 000, et son indice de polydispersité est égal à 1,8.

Selon une autre réalisation, la vis 1 peut être formée d'un matériau composite comprenant une matrice de polymère telle qu'indiquée ci-dessus, et une charge minérale ostéoconductive telle que de l'hydroxyapatite.

En particulier, le pourcentage en poids de charge minérale est compris entre 15 et 50% du poids total du mélange polymère-charge minérale.

Le mélange polymère-charge minérale est de façon avantageuse réalisé en phase solvant, lors de l'étape de précipitation du polymère.

La vis 1 est du type sans tête. Elle est constituée d'un corps 2 monobloc sensiblement cylindrique sur sa majeure partie, et comportant une extrémité 3 de forme tronconique. Ce corps 2 est pourvu d'une surface externe 4 sur laquelle est prévu le filet 5 de la vis 1.
Dans le mode de réalisation représenté, ce filet 5 de vis est prévu sur sensiblement la totalité du corps 2 de la vis 1. Toutefois, d'autres réalisations peuvent être envisagées, dans lesquelles le filet 5 serait prévu sur une partie seulement du corps 2 de la vis.

La vis 1 comprend une première zone dite proximale 6 de diamètre D1, une deuxième zone dite distale 7 de diamètre D2 et une zone intermédiaire 8 située entre la zone proximale 6 et la zone distale 7.

Les termes « proximal » et « distal » sont définis par rapport au sens d'implantation de la vis 1 dans le tunnel trans-osseux.

Selon l'invention, le diamètre D1 est supérieur au diamètre D2 de telle sorte que la vis 1 permette, outre la fixation du tendon, la neutralisation de son mouvement dans le tunnel trans-osseux. La zone de plus grand diamètre est en effet prévue pour comprimer de façon importante le tendon sur la paroi du tunnel, de sorte à obtenir une fixation mécanique optimale. Et la zone de plus faible diamètre est prévue pour neutraliser les mouvements du tendon dans le tunnel.

Selon une réalisation particulière, le diamètre D1 est supérieur au diamètre D2 d'une valeur comprise entre 10% et 20% du diamètre D1. Cet écart entre les diamètres D1 et D2 a été déterminé au cours d'essais réalisés par la demanderesse. Il est en effet apparu qu'un écart inférieur à 10% entre les deux diamètres ne permet pas à la vis de fixer de façon optimale les tendons. Et un écart supérieur à 20% rend la mise en place de la vis par le chirurgien plus difficile.

Selon le mode de réalisation représenté, la différence entre les diamètres D1 et D2 est obtenue, lors de la fabrication de la vis, en prévoyant que la surface externe 4 du corps 2 de la vis 1 ait un diamètre différent en fonction de la zone à laquelle elle appartient, tout en maintenant constante la profondeur du filet 5 quelque soit la zone. Le diamètre de cette surface externe 4, en zone proximale 6, est donc supérieur au diamètre de la surface externe 4 en zone distale 7.

Ce mode de réalisation permet une fixation optimale du tendon lors de la mise en place de la vis 1.

Toutefois, selon une autre réalisation non représentée, la différence entre les diamètres D1 et D2 peut également être obtenue en prévoyant que le diamètre de la surface externe 4 pourvue du filet 5 soit sensiblement constante, et en faisant varier la profondeur du filet 5 en fonction de la zone sur laquelle il s'étend. La profondeur du filet 5 prévu sur la zone proximale 6 est dans ce cas supérieure à la profondeur du filet 5 en zone distale 7.

En outre, le diamètre de la zone intermédiaire 8 entre les zones proximale 6 et distale 7 est prévu pour décroître continûment entre le diamètre D1 de la zone proximale 6 et le diamètre D2 de la zone distale 7.

La longueur de cette zone intermédiaire 8 est prévue pour être relativement courte par rapport à la longueur totale de la vis 1 de sorte à induire, une fois la vis mise en place sur le tendon, une différence importante de compression sur le tendon entre la zone proximale 6 et la zone distale 7. Cette différence de compression a pour but de permettre une fixation optimale. Toutefois, la longueur de la zone intermédiaire 8 ne doit pas être trop faible par rapport à la longueur totale, car cela entraînerait des difficultés de mise en place de la vis dans le tunnel.

Pour répondre à ces contraintes, des essais ont été réalisés. Il est apparu qu'une réalisation avantageuse est celle représentée sur les figures, dans laquelle la longueur de la zone intermédiaire 8 est sensiblement égale à un tour de la vis.

Dans le mode de réalisation représenté, la partie du filet 5 prévue sur la zone proximale 6 présente un profil trapézoïdal, et la partie du filet 5 s'étendant sur la zone distale 7 présente un profil arrondi.

Le premier pas de vis 9 comprend une arête tranchante pour permettre une meilleur amorce de vissage lors de la mise en place de la vis 1. Puis le profil du reste du filet 5 est agencé pour éviter d'endommager les fibres du tendon, le profil trapézoïdal permettant notamment de limiter le glissement des tendons sous le filet 5.

En outre, sur l'extrémité tronconique 3 du corps 2, le filet 5 présente une dimension radiale qui décroît régulièrement jusqu'à être sensiblement nulle, c'est-à-dire qu'elle est agencée pour se fondre dans l'extrémité 10 du corps 2. En particulier, cette décroissance est réalisée sur une longueur sensiblement égale à un demi tour de vis. Grâce à cet agencement, le filet 5 ne présente pas d'arête vive sur l'extrémité 10.

La vis 1 comprend également un alésage axial traversant dont la partie proximale 12 est de forme prismatique, et dont la partie distale 13 est de forme cylindrique.

Cet alésage est destiné à permettre la mise en place de la vis 1 au moyen d'un tournevis comportant une partie de forme prismatique de section légèrement inférieure à celle de la partie prismatique 12 de l'alésage et une extrémité élargie permettant sa préhension et sa manipulation aisées.

Ainsi, une fois que la partie prismatique du tournevis est complètement engagée dans la partie prismatique 12 de la vis 1, le tournevis peut être actionné pour permettre le vissage de la vis 1 à l'intérieur du tunnel trans-osseux.^^

Selon la réalisation représentée, la partie prismatique 12 de la vis 1 représente 80% de la longueur totale de la vis. Ainsi, le couple de vissage est réparti sur la majeure partie de la longueur de la vis.

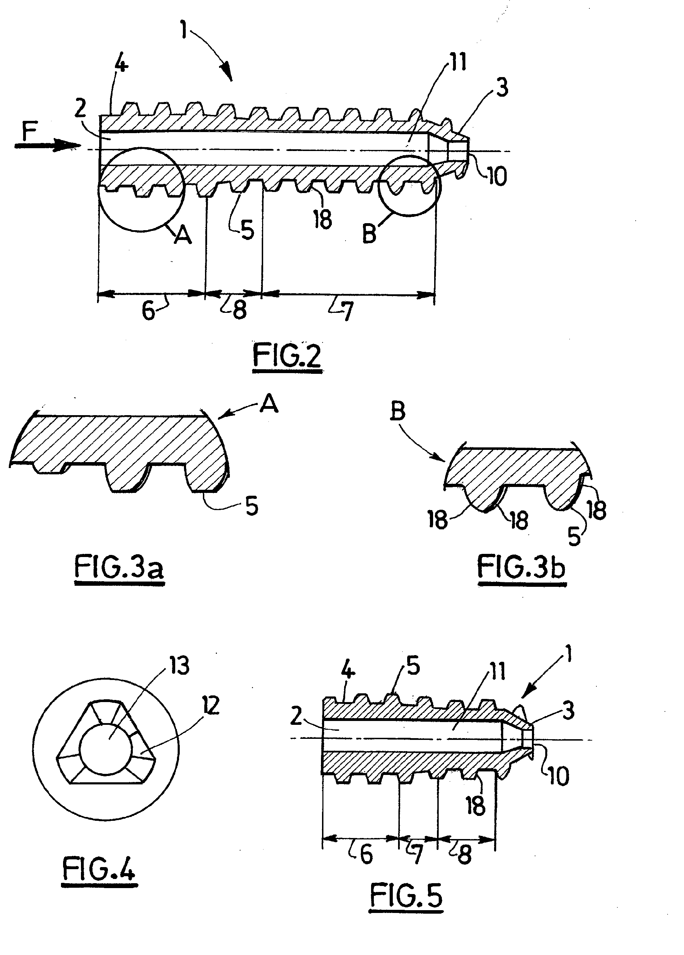

On décrit à présent deux exemples de vis selon l'invention, en relation avec les figures 2 à 5, correspondant respectivement à une vis longue (figures 2-4) et à une vis courte (figure 5).

La vis 1 représentée sur la figure 2 est de longueur totale égale à 30 mm. La longueur de la partie cylindrique du corps 2 est égale à 26 mm, la partie tronconique 3 étant de longueur égale à 4 mm.

Les longueurs des zones proximale 6, intermédiaire 8 et distale 7 sont respectivement égales à 10 mm, 4 mm et 12 mm.

Le diamètre D1 est égal à 9,5 mm, et le diamètre D2 est égal à 8,5 mm. En effet, il a été montré qu'une différence entre ces deux diamètres égale à 1mm permettait d'obtenir des résultats intéressants en termes de fixation et de neutralisation des tendons.

Certaines zones du filet 5 sont représentées de façon agrandie, sur les figures 3a et 3b.

Les deux flancs 18 du filet 5 déterminent entre eux un angle d'environ 30°, et le pas de vis est égal à 3mm.

La vis 1 représentée sur la figure 5 est de longueur totale égale à 20 mm. La longueur de la partie cylindrique du corps 2 est égale à 17 mm, la partie tronconique 3 étant de longueur égale à 3 mm.

Les longueurs des zones proximale 6, intermédiaire 8 et distale 7 sont respectivement égales à 8 mm, 4 mm et 5 mm.

Le diamètre D1 est égal à 9,5 mm, et le diamètre D2 est égal à 8,5 mm.

## Revendications

1. Vis d'interférence (1), notamment destinée à la fixation de tendons à l'intérieur d'un tunnel trans-osseux, ladite vis (1) étant réalisée en matériau biorésorbable et étant constituée d'un corps (2) monobloc pourvu d'une surface externe (4) sur laquelle est prévu un filet (5) de vis, ladite vis (1) comprenant une première zone (6) dite proximale de diamètre D1, une deuxième zone (7) dite distale de diamètre D2 et une zone intermédiaire (8) prévue entre lesdites zones proximale (6) et distale (7), ladite vis (1) étant **caractérisée en ce que** le diamètre D1 est supérieur au diamètre D2 de sorte à permettre, outre la fixation des tendons, la neutralisation de leur mouvement dans les tunnels trans-osseux.

2. Vis selon la revendication 1, **caractérisée en ce que** le diamètre D1 est supérieur au diamètre D2 d'une valeur comprise entre 10% et 20% du diamètre D1.

3. Vis selon la revendication 1 ou 2, **caractérisée en ce que** le diamètre de la surface externe (4) pourvue du filet (5) est sensiblement constante.

4. Vis selon la revendication 1 ou 2, **caractérisée en ce que** la surface externe (4) du corps (2) présente un diamètre différent en fonction de la zone à laquelle elle appartient.

5. Vis selon l'une des revendications 1 à 4, **caractérisée en ce que** le diamètre de la zone intermédiaire (8) décroît continûment entre le diamètre D1 de la zone proximale (6) et le diamètre D2 de la zone distale (7).

6. Vis selon la revendication 5, **caractérisé en ce que** la longueur de la zone intermédiaire (8) est sensiblement égale à un tour de la vis.

7. Vis selon l'une des revendications 1 à 6, **caractérisée en ce que** la partie de filet (5) prévue sur la zone proximale (6) présente un profil trapézoïdal, et la partie de filet (5) s'étendant sur la zone distale (7) présente un profil arrondi.

8. Vis selon l'une des revendications 1 à 7, **caractérisée en ce que** le corps (2) comprend en outre une extrémité (3) de forme tronconique sur laquelle le filet (5) présente une dimension radiale décroissant régulièrement jusqu'à être sensiblement nulle sur l'extrémité (10) du corps (2).

9. Vis selon la revendication 8, **caractérisée en ce que** la décroissance est réalisée sur une longueur sensiblement égale à un demi tour de la vis.

10. Vis selon l'une des revendications 1 à 9, **caractérisée en ce que** le filet (5) est prévu sur sensiblement la totalité du corps (2) de la vis (1), ladite vis (1) étant du type sans tête.

11. Vis selon l'une quelconque des revendications 1 à 10, **caractérisée en ce qu'**elle comprend un alésage axial traversant dont la partie proximale (12) est de forme prismatique et dont la partie distale (13) est de forme cylindrique.

12. Vis selon la revendication 11, **caractérisé en ce que** la partie proximale (12) représente 80% de la longueur totale de la vis.

13. Vis selon l'une quelconque des revendications 1 à 12, **caractérisée en ce qu'**elle est réalisée à partir d'un stéréocopolymère d'acide lactique de haute pureté chimique, comportant au moins 95% de motifs dérivés de l'acide L-lactique, une masse supérieure à 250 000 et un indice de polymolécularité inférieur à 2.

14. Vis selon la revendication 13, **caractérisée en ce qu'**elle comprend en outre une charge minérale ostéoconductive, telle que de l'hydroxyapatite.

15. Vis selon la revendication 14, **caractérisée en ce qu'**elle comprend un pourcentage en poids de charge minérale compris entre 15 et 50% du poids total.
